(19) Europäisches Patentamt
European Patent Office
Office européen des brevets

(11) Veröffentlichungsnummer: **0 316 639 B1**

# (12) EUROPÄISCHE PATENTSCHRIFT

(45) Veröffentlichungstag der Patentschrift: **08.06.94**

(51) Int. Cl.[5]: **C07D 403/04**, A61K 31/50

(21) Anmeldenummer: **88117969.1**

(22) Anmeldetag: **28.10.88**

## (54) Azelastin-Embonat, Verfahren zu seiner Herstellung und pharmazeutische Zubereitungen, die als Wirkstoff Azelastin-Embonat enthalten.

(30) Priorität: **13.11.87 DE 3738641**

(43) Veröffentlichungstag der Anmeldung:
**24.05.89 Patentblatt 89/21**

(45) Bekanntmachung des Hinweises auf die Patenterteilung:
**08.06.94 Patentblatt 94/23**

(84) Benannte Vertragsstaaten:
**AT BE CH DE ES FR GB GR IT LI LU NL SE**

(56) Entgegenhaltungen:
**DE-A- 2 164 058**

(73) Patentinhaber: **ASTA Medica Aktiengesellschaft**
**An der Pikardie 10**
**D-01277 Dresden(DE)**

(72) Erfinder: **Scheffler, Gerhard, Dr.**
**Frankenwaldstrasse 19**
**D-6450 Hanau(DE)**
Erfinder: **Sauerbier, Dieter**
**Ranke Horst 18**
**D-4806 Werther(DE)**
Erfinder: **Engel, Jürgen, Dr.**
**Erlenweg 3**
**D-8755 Alzenau(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Azelastin ist ein Wirkstoff mit antiallergischer und asthmaprophylaktischer Wirkung. Die chemische Bezeichnung ist:

4-(p-Chlor-benzyl)-2- hexahydro-1-methyl-azepin-4-yl - 1-(2H)-phthalazinon (vgl. DE-A- 21 64 058).

Eine wichtige Anwendung von Azelastin als Antiallergikum ist die orale Applikation, insbesondere in Form von Tabletten, Kapseln, Lösungen oder Suspensionen, außerdem auch die Applikation in Form von Aerosolen.

Die Anwendung von Azelastin in Form von Lösungen beziehungsweise Suspensionen ist jedoch bis jetzt nicht möglich, da das Azelastin einen derart durchdringenden bitteren Geschmack besitzt, daß jede orale Applikation solcher Azelastin-Lösungen beziehungsweise Azelastin-Suspensionen von den Patienten abgelehnt wird. Dieser bittere Geschmack konnte auch nicht durch Überführung in die verschiedensten Salze behoben werden.

Es wurde nun gefunden, daß das Azelastin mit Embonsäure in ein Produkt übergeführt werden kann, welches nicht mehr den durchdringenden bitteren Geschmack besitzt und daher für die Verwendung von beispielsweise oral einzunehmenden Zubereitungen geeignet ist. Bei dem Azelastin-Embonat handelt es sich um ein Salz von Azelastin mit der Embonsäure, wobei dieses Salz aus 2 Mol Azelastin und 1 Mol Embonsäure besteht (siehe Beispiel 1).

Das erfindungsgemäße Azelastin-Embonat ist besonders geeignet zur Herstellung von oral einzunehmenden galenischen Zubereitungen des Azelastins in Form von stabilen Suspensionen, beispielsweise in Form eines Saftes. Natürlich kann das erfindungsgemäße Azelastin-Embonat auch für die Herstellung anderer galenischer Zubereitungen des Azelastins verwendet werden wie zum Beispiel von Tabletten, Kapseln oder Sprays.

Falls das erfindungsgemäße Azelastin-Embonat zur Herstellung von wäßrigen Suspensionen verwendet wird, handelt es sich um Suspensionen, die als Wirkstoff 3 bis 3000 mg, vorzugsweise 15 bis 240 mg, insbesondere 60 bis 120 mg Gewichtsteile Azelastin-Embonat, bezogen auf 100 ml Suspension enthalten. Vorzugsweise wird hierbei ein Azelastin-Embonat mit einer Korngröße unter 100 µm eingesetzt. Der pH-Wert einer solchen Suspension liegt in dem Bereich von 3 bis 9, vorzugsweise 5 bis 8, insbesondere 6 bis 7.

Besonders günstig ist eine Azelastin-Embonat-Suspension die aus einem thixotropen System besteht, welches im Ruhezustand eine hohe Viskosität aufweist, deren Gerüst jedoch bei geringer mechanischer Beanspruchung (zum Beispiel beim Ausgießen) zusammenbricht, so daß die Suspension, (zum Beispiel der Saft) fließfähig wird.

Zur Herstellung einer derartigen thixotropen Suspension in Wasser werden Quellmittel verwendet. Als solche Quellmittel kommen beispielsweise in Frage: Natürliche Makromoleküle (zum Beispiel Alginate, Pektine, Tragant, hydrokolloide Polysaccharide wie Xanthan Gummi), halbsynthetische Makromoleküle (zum Beispiel Celluloseäther), synthetische Makromoleküle (zum Beispiel Polyacrylate, Polyvinylpyrrolidon) sowie anorganische Hydrogelbildner (zum Beispiel kolloidale Kieselsäure, Bentonit). Diese Quellmittel können einzeln oder im Gemisch zur Verwendung kommen. Aufgrund ihrer ausgeprägten thixotropen Eigenschaften haben sich Zubereitungen mit Xanthan Gummi als besonders geeignet für die Herstellung stabiler, gießfähiger Suspensionen erwiesen.

Diese Quellmittel können einzeln oder im Gemisch zur Verwendung kommen. Die Gesamtmenge an Quellmittel, bezogen auf 100 ml Suspension, beträgt zum Beispiel 0,1 bis 10, vorzugsweise 0,5 bis 5 g. Bei Verwendung von Xanthan Gummi beträgt die Menge an Xanthan Gummi zum Beispiel 0,1 bis 3, vorzugsweise 0,3 bis 1,5, insbesondere 0,5 bis 1 g, bei Polyacrylaten 0,1 bis 1 g, bei Alginaten und Traganth 0,1 bis 0,2 g, bei Pektinen und Celluloseethern 0,5 bis 5 g, bei Polyvinylpyrrolidon und anorganischen Hydrogelbildnern 1 bis 10 g (jeweils bezogen auf 100 ml Suspension).

Weiterhin enthalten die erfindungsgemäßen Azelastin-Embonat-Suspensionen gegebenenfalls übliche in der Pharmazie, beziehungsweise in der Galenik verwendbare Konservierungsmittel, Süßstoffe, Aromastoffe und Farbstoffe.

Als Konservierungsmittel kommen beispielsweise in Frage: organische Säuren (zum Beispiel Sorbinsäure, Benzoesäure), Phenole (zum Beispiel p-Hydroxybenzoesäureniederalkylester), organische Quecksilberverbindungen (zum Beispiel Thiomersal), quartäre Ammoniumverbindungen (zum Beispiel Benzethoniumchlorid), aromatische und aliphatische Alkohole (zum Beispiel 1,2-Propylenglykol, Benzylalkohol), Chlorhexidin. Die Konservierungsmittel können auch in form ihrer Salze verwendet werden (zum Beispiel Alkalisalze wie Natriumbenzoat) und natürlich auch als Gemische.

Die Menge an Konservierungsmittel in 100 ml Suspension kann beispielsweise liegen für Sorbinsäure bei 0,05 g bis 1,0 g. Benzoesäure 0,1 g bis 0,2 g, Thiomersal 0,001 g bis 0,01 g, Benzethoniumchlorid

0,005 g bis 0,02 g, 1,2-Propylenglykol 10 g bis 30 g, Benzylalkohol 1,0 g bis 2,0 g, Chlorhexidin 0,001 bis 0,01 g.

Bevorzugt wird ein Gemisch von p-Hydroxybenzoesäureniederalkylestern eingesetzt. Die Summe an p-Hydroxybenzoesäureniederalkylestern, bezogen auf 100 ml Suspension, liegt zum Beispiel zwischen 0,1 und 0,3 g vorzugsweise zwischen 0,15 g und 0,25 g, insbesondere zwischen 0,15 g und 0,20 g.

Als Süßstoffe kommen beispielsweise in Frage:
Saccharin, Cyclamat, Aspartam, Fructose, Saccharose, Sorbit, Mannit sowie vorzugsweise Xylit. Die Menge an Süßstoff richtet sich naturgemäß nach dem Süßwert. Im allgemeinen beträgt die Menge, bezogen auf 100 ml Suspension, für Saccharin 0,005 bis 0,1 für Cyclamat 0,5 bis 2,0, für Aspartam 0,005 bis 0,3, für Fructose, Saccharose, Sorbit und Mannit 1,0 bis 60 g.
Für Xylit beträgt diese Menge beispielsweise 1 bis 60, vorzugsweise 15 bis 60, insbesondere 30 bis 40 g.

Als Aromastoffe kommen in Frage:
ätherische Öle (zum Beispiel Pfefferminzöl, Melissenöl, Citronenöl), Fruchtextrakte (zum Beispiel Citrone, Grapefruit, Ananas), aromatische Drogenextrakte (Süßholzwurzel, Anis, Fenchel), naturidentische und synthetische Aromastoffe. Als besonders geeignet erweist sich zum Beispiel Himbeeraroma.
Die Menge an Aromastoffen liegt beispielsweise, bezogen auf 100 ml Suspension, zwischen 0,001 bis 5 oder auch 10, vorzugsweise 0,01 bis 1, insbesondere 0,01 bis 0,1 g.
Für Himberraroma kommen zum Beispiel 0,01 bis 0,1, vorzugsweise 0,01 bis 0,05, insbesondere 0,02 bis 0,04 g pro 100 ml Suspension in Frage.

Als Farbstoff kommen beispielsweise in Frage:
die üblichen zugelassenen Lebensmittelfarbstoffe, Farbstoffe aus natürlichen Lebensmitteln (zum Beispiel Curcumin, Riboflavin, Chlorophyll, Xanthophylle) synthetische organische Farbstoffe (Azofarbstoffe, Azofarblacke), anorganische synthetische Farbstoffe (zum Beispiel Titandioxid, Eisenoxide). Als besonders geeignet erweisen sich synthetische Azofarbstoffe wie zum Beispiel Amaranth.

Die Menge an Farbstoffen kann beispielsweise liegen zwischen 0,001 bis 1,0, vorzugsweise 0,001 bis 0,1, insbesondere 0,001 bis 0,01 g, bezogen auf 100 ml Suspension. Für Amaranth kommen zum Beispiel 1 bis 10, vorzugsweise 1 bis 5, insbesondere 2 bis 4 mg, bezogen auf 100 ml Suspension, in Frage.

Die Einstellung des erforderlichen pH-Wertes erfolgt zweckmäßig mittels anorganischer Säuren (Salzsäure, Schwefelsäure, Phosphorsäure), organischer Säuren (zum Beispiel Citronensäure, Maleinsäure), anorganischer Laugen (zum Beispiel Natronlauge, Kalilauge) oder mittels hierfür üblicher Salze (zum Beispiel Ammoniumchlorid, Natriumcitrat, Natriumdihydrogenphosphat).

Zur Herstellung von Suspensionen des erfindungsgemäßen Azelastin-Embonats können neben Wasser auch andere physiologisch verträgliche Flüssigkeiten eingesetzt werden. Als solche Flüssigkeiten kommen beispielsweise in Frage: Einwertige und mehrwertige niedere Alkohole wie zum Beispiel Ethanol, Propylenglykol, Glycerin und Polyglykole mit Molekulargewichten von 200 bis 600D. Es können auch Gemische dieser Flüssigkeiten miteinander, sowie mit Wasser verwendet werden.
Als flüssige Trägerstoffe kommen auch zum Beispiel in Frage: natürliche Öle (zum Beispiel Olivenöl). sythetische und halbsynthetische ölartige pharmazeutische Trägerflüssigkeiten wie Triglyceride von gesättigten Pflanzensäuren mit 8 bis 12 C-Atomen und deren Gemische.
Vorzugsweise handelt es sich um rein wäßrige Suspensionen.

Falls Gemische aus Wasser und anderen Flüssigkeiten verwendet werden, handelt es sich beispielsweise um Gemische, bei denen der Gehalt an dem nicht wäßrigen Anteil 1 bis 60, vorzugsweise 10 bis 40, insbesondere 20 bis 30 Gewichtsprozent, bezogen auf 100 Gramm Supension, beträgt.

Gegebenenfalls können den erfindungsgemäßen Suspensionen auch Netzmittel zugesetzt werden. Als solche Netzmittel kommen beispielsweise in Frage: Anionische Tenside, zum Beispiel Seifen, Fettalkoholsulfate, nichtionogene Tenside, zum Beispiel Polyethylenglykol-Fettsäureester (Myrj), Polyethylenglykol-Fettalkoholäther (Brij), Sorbitanfettsäureester (Span), Polyethylenglykol-Sorbitanfettsäureester (Tween), Polyethylenglykol-Polypropylenglykolderivate (Pluronics).
Bevorzugt werden Sorbitanfettsäureester (mit gesättigten oder ungesättigten aliphatischen Carbonsäuren von $C_{10}$ bis $C_{20}$), Polyoxyethylenfettalkoholether (Alkohole von $C_{10}$ bis $C_{20}$) und Polyethylenglykol-Sorbitancarbonsäureester (gesättigte oder ungesättigte aliphatische Carbonsäuren von $C_{10}$ bis $C_{20}$) eingesetzt.

Die Menge an Netzmittel, bezogen auf 100 ml Suspension, kann z.B. betragen: 1 bis $10^{-5}$, vorzugsweise 0,5 bis 0,001 insbesondere 0,1 bis 0,01 g. Das Netzmittel hat die Funktion, eine optimale Dispergierung des nicht gelösten Wirkstoffes zu gewährleisten. Gegebenenfalls ist die erforderliche Menge an dem jeweiligen Netzmittel durch Vorversuche zu ermitteln.

Die erfindungsgemäßen Azelastin-Embonat-Suspensionen haben beispielsweise Viskositäten in einem Bereich von 0,05 bis 0,22, vorzugsweise 0,09 bis 0,18, insbesondere 0,12 bis 0,15 Pascal-Sekunde (Pa.s.)

bei einer Schergeschwindigkeit von 110 pro Sekunde im Rotationsviskosimeter.

Darüberhinaus kann den erfindungsgemäßen Azelastin-Embonat-Suspensionen noch zusätzlich Embonsäure zugesetzt werden. Pro 100 ml Suspension können beispielsweise 1 bis 2000 mg, vorzugsweise 20 bis 1000 mg, insbesondere 50 bis 150 mg Embonsäure zusätzlich zu dem bereits vorhandenen Azelastin-Embonat zugesetzt werden.

Die überschüssige Embonsäure bewirkt überraschenderweise eine zusätzliche Geschmacksverbesserung.

Zur Herstellung von Suspensionen mit Treibmitteln (Aerosole) werden zusätzlich beziehungsweise anstelle der genannten Hilfsstoffe die üblichen Treibgase (Propan, butan, Fluorchlorkohlenwasserstoffe) verwendet. Für solche Suspensionen soll das Azelastin-Embonat beispielsweise eine Teilchengröße mit Durchmessern zwischen 5 - 10 $\mu$m aufweisen.

Die Herstellung solcher Aerosole erfolgt zum Beispiel dadurch, daß man bei einer Temperatur zwischen -55 °C und +55 °C 3 bis 3000 mg Azelastin-Embonat in 100 ml eines Gemisches aus chlorierten fluorierten Kohlenwasserstoffen und/oder Kohlenwasserstoffen unter Zusatz von 0,25 bis 3 g Sorbitantrioleat sowie gegebenenfalls weiteren Hilfsstoffen dispergiert und die so erhaltene Suspension in Dosen abfüllt, die mit Dosierventilen verschlossen sind beziehungsweise werden, welche pro Betätigung 0,025 bis 0,1 ml der Suspension freisetzen.

Die Herstellung von Azelastin-Embonat dieser Teilchengröße erfolgt durch Vermahlen in einer üblichen Mikronisiereinrichtung.

Für die Herstellung von anderen oralen Zubereitungen des Azelastin-Embonats werden die üblichen pharmazeutisch und galenisch verwendbaren Hilfs- und Trägerstoffe verwendet. Beispielsweise werden für Tabletten die folgenden Hilfsbeziehungsweise Trägerstoffe verwendet (Menge in Gewichtsprozent pro Tablette).

| | |
|---|---|
| Füllmittel (5-95 %): | zum Beispiel Stärke, Cellulose, Milchzucker, Saccharose, Fructose, Sorbit, Mannit, Calciumphosphat. |
| Bindemittel (1-80 %): | Gelatine, Celluloseether, Pektine, Alginate, Polyvinylpyrrolidon, Lactose, mikrokristalline Cellulose. |
| Sprengmittel (1-10 %): | Alginate, Stärke, Pektine, Carboxymethylcellulose, Polyvinylpolypyrrolidon, Ultramylopektin, Bentonite. |
| Gleitmittel (0,2-10 %); | Stearinsäure, Stearate, Polyglykole, Talkum, hochdisperses Siliciumdioxid. |

Ferner können Tabletten noch enthalten: Gegenklebemittel, Resorptionsbeschleuniger, Hydrophilisierungsmittel, Feuchthaltemittel und äquivalente Mittel. Oftmals werden überzogene Tabletten hergestellt, die beispielsweise dann noch entsprechende Filmbildner und Überzugsmaterialien sowie Farbstoffe, Weichmacher, Poliermittel enthalten.

Die erwähnten Füllmittel, Bindemittel und Gleitmittel können auch bei den anderen oralen Arzneiformen (Kapseln, Granulate und ähnliche) eingesetzt werden.

Tabletten sowie auch andere orale Zubereitungen (Kapseln, Granulate) enthalten beispielsweise zwischen 0,5 bis 30 mg, vorzugsweise 1 bis 20 mg, insbesondere 1,5 bis 12 mg Azelastin-Embonat.

Die Herstellung des erfindungsgemäßen Azelastin-Embonats erfolgt durch Umsetzung von Azelastin beziehungsweise einem Säureadditionsalz des Azelastins mit Embonsäure beziehungsweise einem Salz der Embonsäure in einem geeigneten Lösungsmittel, wobei gegebenenfalls erhitzt wird. Die Umsetzung erfolgt bei Temperaturen von 18 bis 150 °C, insbesondere 20 bis 100 °C, vorzugsweise zwischen 20 bis 50 °C.

Als Lösungsmittel kommen zum Beispiel in Frage: niedere aliphatische $C_1$-$C_6$-Alkohole (Methanol, Ethanol, Propanol, Isopropanol, Butanol), niedere aliphatische Ketone mit 3 bis 8 C-Atomen (Aceton, Methylethyl-keton), Glykolether, cyclische Ether (Dioxan, Tetrahydrofuran), Ester von niederen aliphatischen Carbonsäuren mit niederen aliphatischen Alkoholen, Amide und N-alkylsubstituierte Amide von aliphatischen $C_1$-$C_4$-Carbonsäuren (Dimethylformamid, Dimethylacetamid), $C_1$-$C_6$-Dialkylsulfone (Dimethylsulfon, Tetramethylensulfon), $C_1$-$C_6$-Dialkylsulfoxide (Dimethylsulfoxid) sowie weitere aprotische Mittel wie N-Methylpyrrolidon, Tetra-methylharnstoff, Hexamethylphosphorsäuretriamid, Acetonitril, Gemische dieser Mittel untereinander sowie Gemische mit Wasser. Bei wäßrigen Gemischen ist der Waseranteil im allgemeinen nicht höher als 30 Volumenprozent. Weiterhin kann die Umsetzung auch in Alkohol-Ethergemischen erfolgen, wobei zum Beispiel aliphatische $C_2$-$C_6$-Ether und cyclische Ether verwendet werden können. Ebenfalls ist die Umsetzung möglich in Gemischen von niederen aliphatischen Alkoholen mit halogenierten aliphatischen oder aromatischen Kohlenwasserstoffen.

Azelastin und Embonsäure werden im Verhältnis 2 : 1 eingesetzt. Bevorzugt wird dabei ein Überschuß an Azelastin von 1 - 20 insbesondere 1 - 5 % bezogen auf die Menge Embonsäure, die für das obengenannte Verhältnis notwendig ist, eingesetzt.

Falls das Azelastin in Form eines Salzes eingesetzt wird, kommen zum Beispiel die Salze mit folgenden Säuren in Frage: starke und mittelstarke anorganische Säuren (Halogenwasserstoffsäuren wie HCl, HBr,

Salpetersäure, Phosphorsäuren, Schwefelsäure), starke bis schwach organische Säuren wie aliphatische und aromatische Sulfonsäuren (Methansulfonsäure, Toluolsulfonsäure), aliphatische gesättige und ungesättigte ein- und mehrbasische Carbonsäuren, aromatische Carbonsäuren (Benzoesäure, Toluolcarbonsäure). Die Embonsäure kann ebenfalls in Form eines Salzes eingesetzt werden. Als Salze der Embonsäure kommen zum Beispiel in Frage: Alkalisalze (Na, K, Li). Erdalkalisalze, Magnesiumsalze, Ammoniumsalze, Alkylammoniumsalze.

Die Herstellung der pharmazeutischen Azelastin-Embonat-Zubereitungen erfolgt durch Vermischen beziehungsweise Homogenisieren des Azelastin-Embonats mit den übrigen Hilfs- und Trägerstoffen bei Temperaturen zwischen 15 bis 80, vorzugsweise 18 bis 40, insbesondere 20 bis 30 °C. Zum Zwecke der Reduzierung von Keimen (Sterilisierung) kann gegebenenfalls 15 bis 60 Minuten auf 80 bis 140, vorzugsweise 110 bis 125 °C erhitzt werden.

Bei der Herstellung von Suspensionen kann man beispielsweise wie folgt verfahren: Das Quellmittel (0,1 bis 10, vorzugsweise 0,3 bis 1,5 g pro 100 ml Suspension und gegebenenfalls ein Teil der anderen Hilfsstoffe) werden in Wasser beziehungsweise den anderen aufgeführten Flüssigkeiten oder Gemischen der Flüssigkeiten bei 20 bis 30 °C gelöst, wobei die Wassermenge, beziehungsweise die Menge an Flüssigkeit, so bemessen wird, daß die fertige Suspension 0,03 bis 30, vorzugsweise 0,4 bis 6, insbesondere 0,8 bis 1,2 l Wasser, beziehungsweise Flüssigkeit, bezogen auf 1 g Azelastin-Embonat, enthält. Die so erhaltene wäßrige Lösung kann dann beispielsweise 10 bis 120, vorzugsweise 15 bis 60 Minuten, auf 80 bis 134 °C, vorzugsweise 20 bis 30 Minuten, auf 110 bis 121 °C erhitzt werden.
Nach Abkühlen auf 25 bis 35 °C werden zu dieser Lösung gegebenenfalls ein Netzmittel und anschließend das bei Temperaturen von 20 bis 30 °C hergestellte Gemisch aus dem Azelastin-Embonat und gegebenenfalls die Konservierungsstoffe, Süßstoffe, Farbstoffe sowie gegebenenfalls Aromastoffe und weitere Hilfs-und /oder Trägerstoffe zugegeben und das Ganze homogenisiert (Temperatur 15 bis 35, vorzugsweise 20 bis 30 °C).
Anschließend erfolgt gegebenenfalls Zusatz von Aromastoffen sowie Einstellung des pH-Wertes auf 3 bis 9.

Bei der Herstellung der obenangegebenen Azelastin-Embonat-Suspension werden beispielsweise auf 1 Gramm Azelastin-Embonat verwendet:
0,005 bis 600 g, vorzugsweise 300 g bis 400 g Süßstoff
0,01 bis 10 g, vorzugsweise 0,2 bis 0,4 g Aromastoff
Ein Teil der hier angegebenen Aromastoff-Menge kann gegebenenfalls auch später zu der Suspension gegeben werden.

Beispiel 1:

Azelastin-Embonat

2 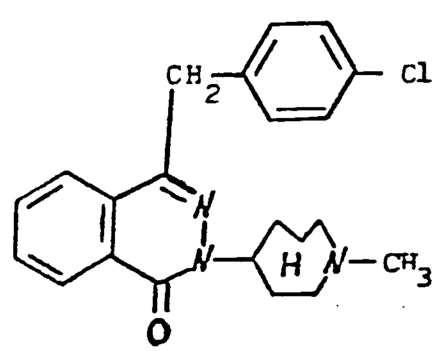 x 1 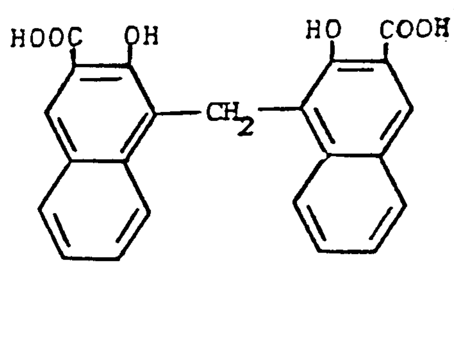

Azelastin (2 Mol) — Embonsäure (1 Mol)

In einem Becherglas werden 177,5 g(1,01 x 2 x 0,21 mol) Azelastin-Hydrochlorid in 4500 ml Ethanol 80%ig durch Umrühren gelöst. Nach Zugabe von 90,6 g (0,21 mol) Embonsäure-Dinatriumsalz wird nur solange weitergerührt, bis sich dieses gelöst hat, circa 4 Minuten.

Danach wird sofort durch ein Faltenfilter filtriert und das Filtrat ruhig über Nacht stehen gelassen. Das Azelastin-Embonat fällt schnell aus. Dieses wird abgesaugt, mit 80%igem Ethanol, dann mit reinem Ethanol

gewaschen und 20 Stunden im Vakuum bei 60 °C getrocknet. Ausbeute 195 g (80 % der Theorie).

Das so erhaltene Produkt wird zur weiteren Reinigung 5 Stunden in Eis-Wasser gerührt, abgesaugt, zuerst mit Eiswasser, dann mit Ethanol gewaschen und im Vakuum bei 60 °C während 20 Stunden getrocknet. Ausbeute 195 g (80 % der Theorie). Das Azelastin-Embonat wird in Form eines kristallinen, schwach gelblich gefärbten Pulvers erhalten.

Schmelzpunkt: 197 bis 201 ° C.

Das IR-Spektrum wird durch Figur 1 wiedergegeben.

Kernmagnetisches Resonanzspektrum siehe Figur 2

Beispiel 2:

Azelastin-Embonat-Suspension

3000 ml Suspension entsprechend 3300 g enthalten:

| | |
|---|---|
| Azelastin-Embonat | 3,600 g |
| Xanthan Gummi | 21,000 g |
| Xylit | 1 200,000 g |
| Natriumpropyl-4-hydroxybenzoat (Na-Salz des 4-Hydroxy-benzoesäurepropylesters) | 1,200 g |
| Natriummethyl-4-hydroxybenzoat (Na-Salz des 4-Hydroxy-benzoesäuremethylesters) | 4,200 g |
| Salzsäure 1 N | 21,000 g (a) |
| Himbeeraroma | 0,900 g |
| Amaranth (zugelassener roter Farbstoff) | 0,150 g |
| Gereinigtes Wasser | 2 047,950 g |
| | 3 300,000 g |

a) Die Salzsäure wird zur Einstellung des pH-Wertes auf 6,5 benötigt. Ein gegenüber dem angegebenen Wert abweichender Einsatz von Salzsäure wird durch entsprechenden Mindereinsatz von gereinigtem Wasser ausgeglichen.

Herstellung:

800,0 g Xylit und 21,0 g Xanthan Gummi werden in 2000 g Wasser in einem 3000 ml Becherglas unter Rühren gelöst. Die Lösung wird anschließend 30 Minuten bei 115 °C im Autoklav erhitzt. Nach dem Abkühlen auf circa 40 °C wird die Lösung unter Vakuum und Rezirkulation in den Arbeitsbehälter eines Homogenisiergerätes gesaugt.

400,0 g Xylit, 1,2 g Natriumpropyl-4-hydroxybenzoat, 4,2 g Natriummethyl-4-hydroxybenzoat, 0,15 g Amaranth und 3,6 g Azelastin-Embonat werden in einer Porzellanschale gemischt und zu der zuvor hergestellten Lösung in den Arbeitsbehälter des Homogenisiergerätes eingesaugt.

0,9 g Himbeeraroma und 21,0 g Salzsäure werden unter Vakuum und Rezirkulation in den Arbeitsbehälter des Homogenisiergerätes eingesaugt. Die Suspension wird 15 Minuten lang homogenisiert.

Der pH-Wert der so erhaltenen Suspension wird durch Zugabe von Salzsäure auf 6,5 eingestellt. Der Verbrauch an Salzsäure wird durch Mindereinsatz von gereinigtem Wasser ausgeglichen. Formel zur Berechnung der benötigten Wassermenge:

47,95 g - eingesetzte Salzsäure in g = Wassermenge in g

Die so erhaltene Suspension ist ein Viskoser, rotgefärbter, Saft (pH-Wert 6,3 bis 6,7).

Wirksamer Bestandteil pro 100 ml: 0,1200 g Azelastin-Embonat

Geruch: Nach Himbeeren

Geschmack: Himbeeraroma

Viskosität: 0,1 - 0,15 Pascal-sekunde (Pa.s.)

Der Saft (Bulkware) wird beispielsweise in Flaschen aus braunem Glas mit Schraubverschluß abgefüllt. Das Abfüllen der Bulkware soll in der Weise geschehen, daß sich keine Lufteinschlüsse durch zu schnelles Ausgießen bilden.

Die Lagerung des Saftes erfolgt beispielsweise bei Raumtemperatur.

Beispiel 3:

Azelastin-Embonat-Suspension

5000 ml Suspension entsprechend 5500 g enthalten:

| | |
|---|---|
| Azelastin-Embonat | 6,000 g (1) |
| Xanthan Gummi | 32,500 g |
| Xylit | 1 500,000 g |
| Natriumpropyl-4-hydroxybenzoat | 2,000 g |
| Natriummethyl-4-hydroxybenzoat | 7,000 g |
| Embonsäure | 5,000 g |
| Himbeeraroma | 1,500 g |
| Amaranth (roter Farbstoff) | 0,250 g |
| Citronensäure | 64,000 g |
| Natriumhydroxid | 32,500 g |
| Gereinigtes Wasser | 3 849,250 g (2) |
| | 5 500,000 g |

1) Das Azelastin-Embonat wurde vor der Verarbeitung durch ein Sieb der Maschenweite 100 µm gegeben.
2) Der pH-Wert der Suspension wird gegebenenfalls mit 1 N Natronlauge auf 6,5 eingestellt. Der Verbrauch an Natronlauge wird beim Wasser in Abzug gebracht.

**Herstellungsgang**

I. 400 g Xylit und 32,5 g Xanthan Gummi werden miteinander verrieben und dieses Gemisch in 3000 g Wasser unter Rühren gelöst. Die Lösung wird 30 Minuten bei 115°C im Autoklav erhitzt. Das beim Autoklavieren verdunstete Wasser wird ergänzt.Nach dem Abkühlen auf ca. 30°C wird dann die Lösung unter Vakuum und Rezirkulation in den Arbeitsbehälter eines Homogenisiergerätes überführt.
II. In der angegebenen Reihenfolge werden 64 g Citronensäure, 5 g Embonsäure, 2 g Natriumpropyl-4-hydroxybenzoat, 7 g Natriummethyl-4-hydroxybenzoat. 0,25 g Amaranth, 6 g Azelastinembonat, 1,5 g Himbeeraroma und 1100 g Xylit unter Vakuum und Rezirkulation in den Arbeitsbehälter des Homogenisiergerätes eingesaugt. Gegebenenfalls wird der pH-Wert mit 1 N Natronlauge auf 6,5 eingestellt.

Mit Wasser wird nachgespült und auf das endgültige Volumen von 5000 ml aufgefüllt. Die Supension wird 15 Minuten lang unter Vakuum und Rezirkulation homogenisiert.

Die so erhaltene Supension ist ein viskoser, roter Saft.

| | |
|---|---|
| Viskosität | = 0,1 - 0,15 Pascal sekunde (Pa.s.) |
| pH-Wert | = 6,3 - 6,7 |
| Dichte | = 1,09 - 1,11 g/ml |

Geruch: Nach Himbeeren
Geschmack: Himbeeraroma

**Patentansprüche**

1. Azelastin-Embonat

2. Pharmazeutische Zubereitungen, die als Wirkstoff Azelastin-Embonat, gegebenenfalls mit weiteren üblichen physiologisch verwendbaren Hilfsstoffen, Trägerstoffen und/oder Verdünnungsmitteln, enthalten.

3. Stabile wäßrige Suspension oder Aerosol, enthaltend als Wirkstoff 3 bis 3000 mg Gewichtsteile Azelastin-Embonat bezogen auf 100 ml Suspension, mit einem pH-Wert von 3 bis 9.

4. Verfahren zur Herstellung von Azelastin-Embonat, dadurch gekennzeichnet, daß man 1 Mol Embonsäure (die zuch in Form eines Salzes vorliegen kann) mit 2 bis 2,4 Mol Azelastin oder 2 bis 2,4 Mol eines Säureadditionssalzes des Azelastins in einem hydrophilen Lösungsmittel, beziehungsweise Lösungsmittelgemisch, Alkohol-Ether-Gemisch oder Alkohol-Halogenkohlenwasserstoff-Gemisch bei einer Temperatur von 18 bis 150 °C umsetzt.

5. Verfahren zur Herstellung von pharmazeutischen Zubereitungen enthaltend Azelastin-Embonat, dadurch gekennzeichnet, daß das Azelastin-Embonat bei einer Temperatur zwischen 15 und 80 °C mit pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird, wobei feste Zubereitungen in der Dosierungseinheit 0,5-30 mg Azelastin-Embonat und Suspensionen 3 bis 3000 mg Gewichtsteile Azelastin-Embonat in 100 ml Suspension enthalten.

6. Verfahren zur Herstellung einer Suspension enthaltend als Wirkstoff Azelastin-Embonat, dadurch gekennzeichnet, daß man bei einer Temperatur zwischen 15 bis 80 °C 3 bis 3000 mg Azelastin-Embonat mit Quellmitteln, gegebenenfalls Netzmitteln, Konservierungsstoffen, Süß- und Aromastoffen sowie Farbstoffen in Wasser oder Gemischen aus Wasser und anderen mit Wasser michschbaren physiologisch verträglichen Flüssigkeiten homogenisiert, wobei die so entstandene Suspension die oben angegebene Azelastin-Embonat-Menge in 100 ml Suspension enthält, und gegebenenfalls die Suspension auf einen pH-Wert zwischen 3 und 9 einstellt.

7. Verfahren zur Herstellung einer wäßrigen Suspension enthaltend als Wirkstoff Azelastin-Embonat, dadurch gekennzeichnet, daß man bei einer Temperatur zwischen 15 bis 80 °C eine homogene Mischung herstellt, die 3 bis 3000 mg Azelastin-Embonat (pro 100 ml Suspension) enthält sowie Konservierungsmittel, Süßstoffe, Farbstoffe und Aromastoffe, wobei auf 100 ml Suspension 0,001 bis 30 g Konservierungsmittel (Gesamtmenge), 0,005 bis 60 g Süßstoff, 0,001 bis 1,0 g Farbstoff sowie 0,001 bis 10 g Aromastoff verwendet werden und diese Mischung bei einer Temperatur zwischen 15 und 80 °C mit einer Lösung von 0,1 bis 10 g Quellmittel in Wasser (bezogen auf 100 ml End-Suspension), wobei bis zu 60 Gewichtsprozent des Wassers durch andere mit Wasser mischbare physiologisch verträgliche Flüssigkeiten ersetzt sein können, homogenisiert, gegebenenfalls die so erhaltene Suspension durch Zusatz von Säure, Lauge oder sauren Salzen auf einen pH-Wert von 3 bis 9 einstellt und gegebenenfalls den Restteil von noch nicht vollständig zugesetzten Aromastoffen und/oder Süßstoffen zusetzt.

8. Verfahren zur Herstellung einer Suspension, enthaltend als Wirkstoff Azelastin-Embonat nach Anspruch 7, dadurch gekennzeichnet, daß 1 bis $10^{-5}$g Netzmittel (bezogen auf 100 ml End-Suspension) während der Suspensions-Herstellung zugegeben werden.

9. Verfahren zur Herstellung einer Suspension, enthaltend als Wirkstoff Azelastin-Embonat, dadurch gekennzeichnet, daß man bei einer Temperatur zwischen -55 °C und +55 °C 3 bis 3000 mg Azelastin-Embonat in 100 ml eines Gemisches aus chlorierten fluorierten Kohlenwasserstoffen und/oder Kohlenwasserstoffen unter Zusatz von 0,25 bis 3 g Sorbitantrioleat sowie gegebenenfalls weiteren Hilfsstoffen dispergiert und die erhaltene Suspension in Dosen abfüllt, die mit Dosierventilen verschlossen sind beziehungsweise werden, welche pro Betätigung 0,025 bis 0,1 ml der Suspension freisetzen.

10. Verfahren zur Herstellung einer festen Zubereitung, enthaltend als Wirkstoff 0,5-30 mg Azelastin-Embonat in der Dosierungseinheit, dadurch gekennzeichnet, daß man das Azelastin-Embonat mit mindestens einem der Hilfsstoffe Stärke, Cellulose, Celluloseether, Zucker, Hexit, Calciumhydrogenphosphat, Calciumphosphat, modifizierte Stärke, Alginat, Pektin, Carboxymethylcellulose, Ultraamylopektin, Bentonit, Polyvinylpyrrolidon vermischt, mit einer wässrigen Gelatinelösung oder Stärkelösung oder einem wässrigen Vinylpyrrolidon-Vinylacetat Copolymerisat granuliert und das erhaltene Granulat gegebenenfalls unter Zusatz von Stearaten, Stearinsäure, Talkum, Polyglykolen und/oder Siliziumdioxid sowie gegebenenfalls auch Stärke und/oder Cellulose zu Tabletten verpresst oder in Kapseln abfüllt, oder gegebenenfalls nach Zusatz von Sojalecitin, bei Temperaturen zwischen 33 bis 37 °C in geschmolzenem Hartfett suspendiert oder homogenisiert und anschließend die Mischung in Hohlzellen ausgießt, wobei den festen Zubereitungen zusätzlich während des Herstellungsprozesses gegebenenfalls auch übliche Gegenklebmittel, Resorptionsbeschleuniger, Hydrophilierungsmittel und/oder Feuchthaltemittel zugesetzt werden können.

**11.** Verwendung von Azelastin-Embonat zur Herstellung von Arzneimitteln.

**Claims**

**1.** Azelastine embonate.

**2.** Pharmaceutical preparations which contain azelastine embonate as active substance optionally with other conventional physiologically acceptable auxiliary substances, carriers and/or diluents.

**3.** A stable aqueous suspension or aerosol containing 3 to 3000 mg parts by weight of azelastine embonate as active substance per 100 ml of suspension at a pH of 3 to 9.

**4.** A process for the preparation of azelastine embonate, characterised in that 1 mole of embonic acid (which may also be present in the form of a salt) is reacted with 2 to 2.4 moles of azelastine or 2 to 2.4 moles of an acid addition salt of azelastine in a hydrophilic solvent, or mixture of solvents, an alcohol/ether mixture or an alcohol/halogenated hydrocarbon mixture at a temperature of 18 to 150 °C.

**5.** A process for the preparation of pharmaceutical preparations containing azelastine embonate, characterised in that the azelastine embonate is processed with pharmaceutical carriers or diluents or other auxiliary substances at a temperature between 15 and 80 °C to produce pharmaceutical preparations or is made into a therapeutically applicable form, wherein solid preparations contain 0.5 - 30 mg of azelastine embonate in the dosage unit and suspensions contain 3 to 3000 mg parts by weight of azelastine embonate in 100 ml of suspension.

**6.** A process for the preparation of a suspension containing azelastine embonate as active substance, characterised in that 3 to 3000 mg of azelastine embonate is homogenised with swelling agents, optional wetting agents, preservatives, sweeteners and flavourings, as well as colorants in water or mixtures of water and other physiologically acceptable liquids which are miscible with water at a temperature between 15 and 80 °C, wherein the resultant suspension contains the amount of azelastine embonate given above in 100 ml of suspension, and optionally the suspension is adjusted to a pH between 3 and 9.

**7.** A process for the preparation of an aqueous suspension containing azelastine embonate as active substance, characterised in that a homogeneous mixture is prepared at a temperature between 15 and 80 °C, this containing 3 to 3000 mg of azelastine embonate (per 100 ml of suspension) as well as preservatives, sweeteners, colorants and flavourings, wherein 0.001 to 30 g of preservatives (total amount), 0.005 to 60 g of sweetener, 0.001 to 1.0 g of colorant and 0.001 to 10 g of flavouring are used per 100 ml of suspension and this mixture is homogenised at a temperature between 15 and 80 °C with a solution of 0.1 to 10 g of swelling agent in water (per 100 ml of final solution), wherein up to 60 percent by weight of the water may be replaced by other physiologically acceptable liquids which are miscible with water, optionally the suspension obtained in this way is adjusted to a pH of 3 to 9 by the addition of acid, an alkaline solution or acid salts and optionally the remainder of the flavourings and/or sweeteners which has not already been added is added.

**8.** A process for the preparation of a suspension containing azelastine embonate as active substance according to Claim 7, characterised in that 1 to $10^{-5}$ g of wetting agent (per 100 ml of final solution) are added during preparation of the suspension.

**9.** A process for the preparation of a suspension containing azelastine embonate as active substance, characterised in that 3 to 3000 mg of azelastine embonate is dispersed in 100 ml of a mixture of chlorinated fluorinated hydrocarbons and/or hydrocarbons, at temperatures between -55 °C and +55 °C, with the addition of 0.25 to 3 g of sorbitan trioleate and optionally other auxiliary substances,

and the resultant suspension is dispensed into cans which are or will be sealed with metering valves which release 0.025 to 0.1 ml of suspension each time they are activated.

**10.** A process for the preparation of a solid preparation, containing 0.5 - 30 mg of azelastine embonate as active substance in the dosage unit, characterised in that the azelastine embonate is mixed with at least one of the auxiliary substances starch, cellulose, cellulose ether, sugar, hexitol, calcium hydrogen phosphate, calcium phosphate, modified starch, alginate, pectin, carboxymethyl cellulose, ultraamylopectin, bentonite, polyvinylpyrrolidone, granulated with an aqueous gelatine solution or starch solution or an aqueous vinylpyrrolidone/vinylacetate copolymer and the granulate obtained is compressed into tablets optionally with the addition of stearates, stearic acid, talcum, polyglycols and/or silicon dioxide and optionally also starch and/or cellulose or capsules are filled therewith, or optionally after the addition of soybean lecithin is suspended or homogenised in molten hard fat at temperatures between 33 and 37 °C and then the mixture is poured into hollow cells, wherein optionally in addition conventional anti-adhesion agents, absorption accelerators, hydrophilising agents and/or humectants may also be added to the solid preparations during the preparation process.

**11.** Use of azelastine embonate for the preparation of medicaments.

**Revendications**

**1.** Embonate d'azélastine.

**2.** Préparations pharmaceutiques, contenant comme principe actif de l'embonate d'azélastine, éventuellement avec d'autres adjuvants, excipients et/ou diluants usuels, physiologiquement utilisables.

**3.** Suspension aqueuse stable ou aérosol contenant comme principe actif de 3 à 3000 mg en poids d'embonate d'azélastine, pour 100 ml de suspension, avec un pH de 3 à 9.

**4.** Procédé pour préparer de l'embonate d'azélastine, caractérisé en ce qu'on fait réagir 1 mole d'acide embonique (qui peut aussi se présenter sous forme d'un sel) avec 2 à 2,4 moles d'azélastine ou 2 à 2,4 moles d'un sel d'addition avec un acide de l'azélastine, dans un solvant hydrophile ou un mélange de solvants, un mélange d'alcool et d'éther ou un mélange d'alcool et d'un hydrocarbure halogéné, à une température de 18 à 150 °C.

**5.** Procédé pour fabriquer des préparations pharmaceutiques contenant de l'embonate d'azélastine, caractérisé en ce qu'on transforme en préparations pharmaceutiques, ou encore en une forme thérapeutiquement utilisable, l'embonate d'azélastine à une température comprise entre 15 et 80 °C, avec des excipients ou diluants pharmaceutiques, ou encore d'autres adjuvants, les préparations solides contenant dans l'unité posologique de 0,5 à 30 mg d'embonate d'azélastine et les suspensions contenant de 3 à 3000 mg en poids d'embonate d'azélastine pour 100 ml de suspension.

**6.** Procédé pour fabriquer une suspension contenant comme principe actif de l'embonate d'azélastine, caractérisé en ce qu'on homogénéise, à une température de 15 à 80 °C, de 3 à 3000 mg d'embonate d'azélastine avec des agents gonflants, éventuellement des mouillants, des conservateurs, des édulcorants et des aromatisants, ainsi que des colorants, dans de l'eau ou des mélanges d'eau et d'autres liquides miscibles à l'eau et physiologiquement compatibles, la suspension ainsi obtenue contenant la quantité ci-dessus d'embonate d'azélastine dans 100 ml de suspension, et éventuellement on ajuste le pH de la suspension à une valeur comprise entre 3 et 9.

**7.** Procédé pour fabriquer une suspension aqueuse contenant comme principe actif de l'embonate d'azélastine, caractérisé en ce que, à une température de 15 à 80 °C, on prépare un mélange homogène contenant de 3 à 3000 mg d'embonate d'azélastine (pour 100 ml de suspension), ainsi que des conservateurs, des édulcorants, des colorants et des aromatisants, où on utilise, pour 100 ml de suspension, de 0,001 à 30 g de conservateur (quantité totale), de 0,005 à 60 g d'un édulcorant, de 0,001 à 1,0 g d'un colorant, et de 0,001 à 10 g d'un aromatisant, ce mélange étant homogénéisé à une température de 15 à 80 °C avec une solution aqueuse de 0,1 à 10 g d'agent gonflant (pour 100 ml de suspension finale), auquel cas on peut remplacer jusqu'à 60 % en poids de l'eau par d'autres liquides miscibles à l'eau et physiologiquement compatibles, éventuellement on ajuste à un pH de 3 à 9 la

suspension ainsi obtenue, par addition d'un acide, d'un base ou de sels acides, et éventuellement on ajoute la partie résiduelle d'aromatisants et/ou d'édulcorants non encore complètement ajoutés.

**8.** Procédé pour préparer une suspension, contenant comme principe actif de l'embonate d'azélastine selon la revendication 7, caractérisé en ce qu'on ajoute pendant la préparation de la suspension de 1 à $10^{-5}$ g d'un mouillant (pour 100 ml de suspension finale).

**9.** Procédé pour préparer une suspension contenant comme principe actif de l'embonate d'azélastine, caractérisé en ce que, à une température comprise entre -55 et +55 °C, on disperse de 3 à 3000 mg d'embonate d'azélastine dans 100 ml d'un mélange d'hydrocarbures chlorés et fluorés et/ou d'hydrocarbures, par addition de 0,25 à 3 g de trioléate de sorbitol et éventuellement d'autres adjuvants, la suspension obtenue étant transvasée dans des boîtes, qui sont déjà obturées ou que l'on obture à l'aide de valves doseuses, qui libèrent à chaque manoeuvre de 0,025 à 0,1 ml de la suspension.

**10.** Procédé pour fabriquer une préparation solide contenant comme principe actif de 0,5 à 30 mg d'embonate d'azélastine dans l'unité posologique, caractérisé en ce qu'on mélange à l'embonate d'azélastine au moins l'un des adjuvants amidon, cellulose, éther de cellulose, sucre, hexitol, hydrogénophosphate de calcium, phosphate de calcium, amidon modifié, alginate, pectine, carboxyméthylcellulose, ultraamylopectine, bentonite, polyvinylpyrrolidone, qu'on le granule avec une solution aqueuse de gélatine ou d'amidon, ou avec un copolymère vinylpyrrolidone-acétate de vinyle aqueux, et on comprime pour obtenir des comprimés ou on introduit dans des gélules le granulé obtenu, éventuellement en ajoutant des stéarates, de l'acide stéarique, du talc, des polyglycols et/ou du dioxyde de silicium et éventuellement aussi de l'amidon et/ou de la cellulose, ou encore, éventuellement après addition de lécithine du soja, on le met en suspension ou on l'homogénéise dans de la graisse dure fondue à des températures de 33 à 37 °C, puis on verse le mélange dans des alvéoles creux, les préparations solides pouvant éventuellement aussi, pendant les opérations de fabrication, étre en outre additionnées d'agents anti-adhésifs, d'accélérateurs de résorption, d'agents d'hydrophilisation et/ou d'humectants usuels.

**11.** Utilisation d'embonate d'azélastine pour préparer des médicaments.

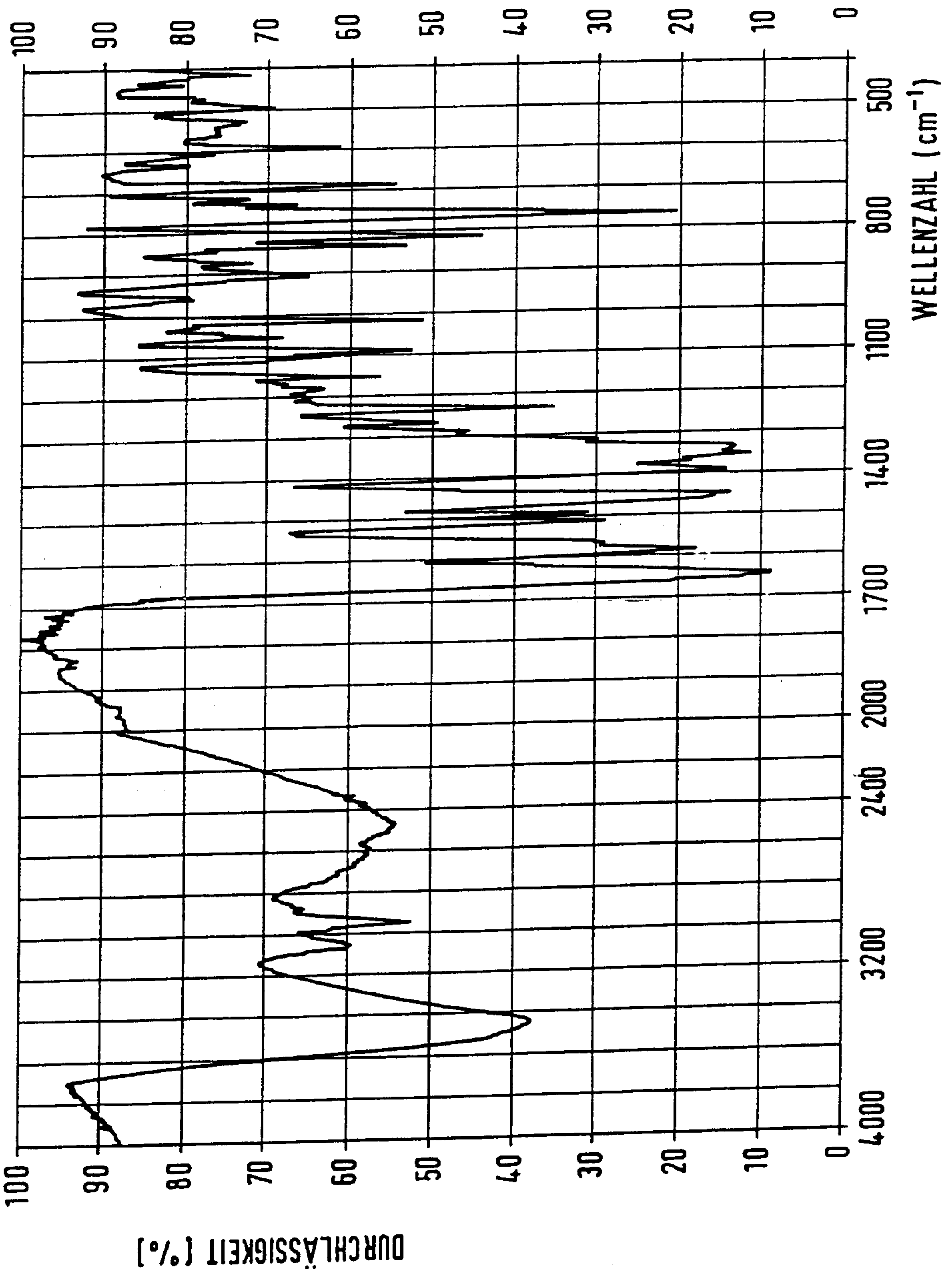


Fig. 1 IR-SPEKTRUM DER AZELASTIN-EMBONAT-SUBSTANZ

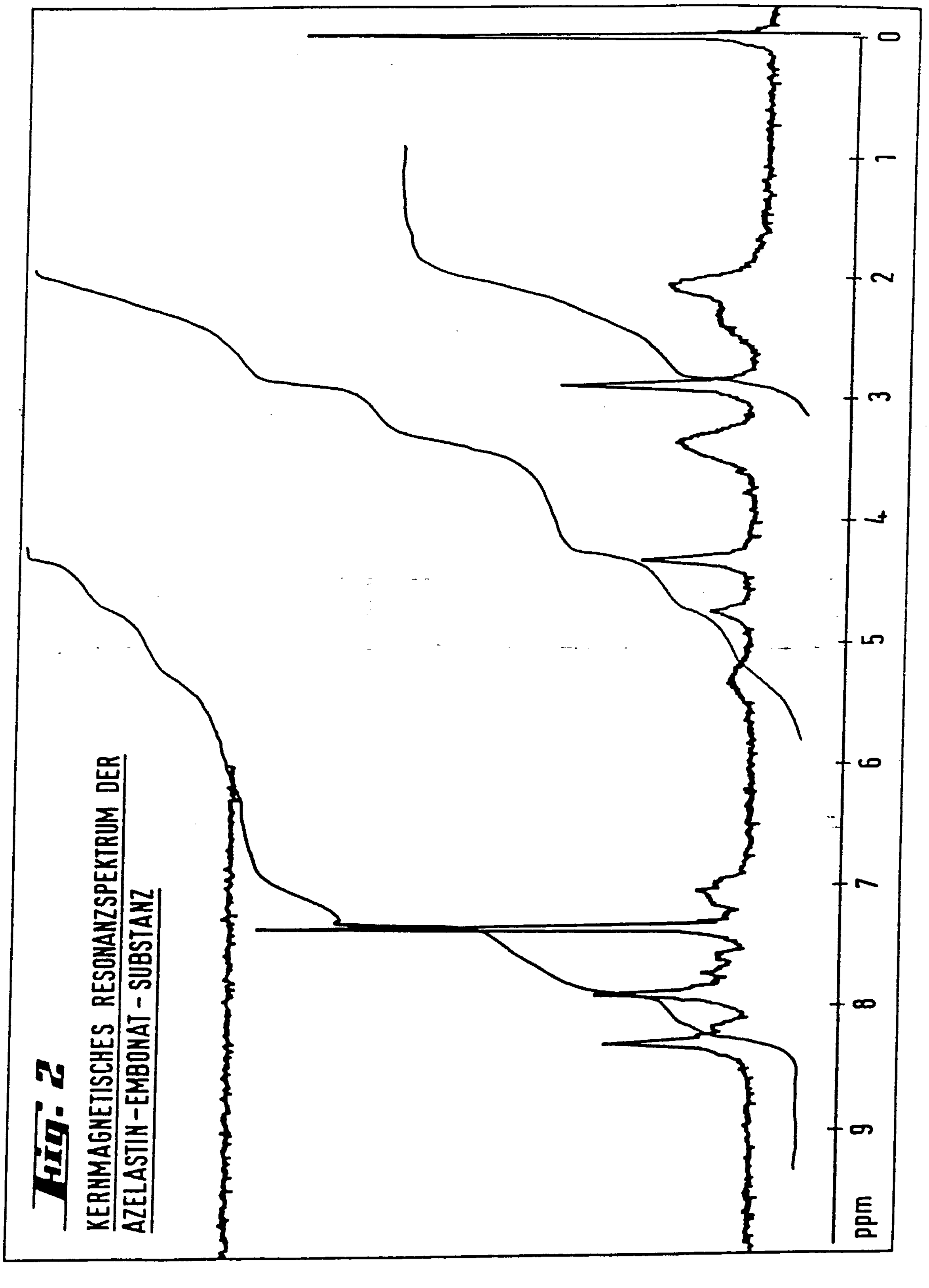


Fig. 2
KERNMAGNETISCHES RESONANZSPEKTRUM DER AZELASTIN-EMBONAT-SUBSTANZ